# EUROPÄISCHE PATENTANMELDUNG

(11) **EP 3 015 542 A1**
(43) Veröffentlichungstag der Anmeldung: **04.05.2016**
(21) Anmeldenummer: 15166686.4
(22) Anmeldetag: 07.05.2015
(51) Int. Cl.: C12M 1/00, C12M 3/00, B01D 15/18, C07K 1/36, C12M 1/12

(54) **MODULARE ANLAGE UND VERFAHREN ZUR KONTINUIERLICHEN, KEIMREDUZIERTEN PRODUKTION UND/ODER AUFBEREITUNG EINES PRODUKTES**

(71) Anmelder: Bayer Technology Services GmbH, 51368 Leverkusen (DE)
(72) Erfinder: Schwan, Peter, 51368 Leverkusen (DE); Maiser, Benjamin, 51375 Leverkusen (DE); Möhrle, Volker, 50737 Köln (DE); Lobedann, Martin, 51069 Köln (DE)
(74) Vertreter: BIP Patents

(57) **Zusammenfassung**

Die Erfindung eines Verfahrens zur kontinuierlichen, keimreduzierten Produktion und/oder Aufbereitung eines biopharmazeutischen, biologischen makromolekularen Produktes aus einer heterogenen Zellkultur-Fluid-Mischung zur Verfügung, umfassend die Schritte: (a) Bereitstellen eines partikel-freien Fluids aus einer heterogenen Zellkultur-Fluid-Mischung, welche das Produkt enthält, in Form eines Produktstroms, (b) mindestens eine Filtration, wobei ein Filtrat erhalten wird, (c) wenigstens zwei Chromatographieschritte zur Reinigung des Produktes, (d) mindestens eine Virenabreicherung, (e) mindestens eine Ultrafiltration und/oder mindestens eine Diafiltration des Produktstroms der Schritte (b), (c), und/oder (d), dadurch gekennzeichnet, dass die wenigstens zwei Chromatographieschritte aus (c) eine Reinigung über wenigstens jeweils zwei Chromatographiesäulen und/oder Membranadsorber umfasst und, dass das Verfahren geschlossen und modular durchgeführt wird. Die Erfindung stellt weiterhin eine entsprechende modulare Anlage zur Durchführung dieses Verfahrens zur Verfügung.

## Beschreibung

Die Erfindung betrifft eine modulare Anlage und ein Verfahren zur kontinuierlichen, keimreduzierten Produktion und/oder Aufbereitung eines Produktes aus einer heterogenen Zellkultur-Fluid-Mischung.

Gewöhnlich werden Proteine in der biotechnologischen Herstellung im Batch aufgereinigt (englisch *batch*: "Stapel"). Das bedeutet, dass die einzelnen Produktionszyklen chargenweise, diskontinuierlich gehandhabt werden, wobei nach dem Abschluss eines Produktionszyklus das Produkt alles Ganzes entnommen wird. Für eine erneute Produktion muss dann separat ein neuere Produktzyklus bzw. Batch gestartet werden.

In den letzten Jahren hat sich nun mehr und mehr gezeigt, dass eine kontinuierliche Verfahrensweise auch in der biotechnologischen Herstellung gefahren werden kann, wobei das Verfahren im Gegensatz zum Batch-Verfahren ohne Unterbrechungen laufen kann.

Die stark regulierte pharmazeutische Produktion erfordert einen großen zeitlichen, technischen und personellen Aufwand für die Bereitstellung gereinigter und sterilisierter Bioreaktoren und Gewährleistung eines keimfreien Produkts. Um Kreuzkontaminationen bei einem Produktwechsel in einer Multi-Purpose-Anlage oder zwischen zwei Produktchargen sicher zu vermeiden, wird außer der Reinigung eine sehr aufwändige Reinigungsvalidierung benötigt, welche bei einer Prozessadaption ggf. wiederholt werden muss.
Dies gilt sowohl für das Upstream-Processing (USP), d.h. die Herstellung biologischer Produkte in Fermentern als auch für das Downstream-Processing (DSP), d. h. die Aufreinigung der Fermentationsprodukte.
Gerade bei der Fermentation ist eine keimfreie Umgebung für eine erfolgreiche Kultivierung essentiell. Zur Sterilisation von Batch- oder Fedbatch-Fermentern kommt in der Regel die SIP-Technik zum Einsatz (SIP = sterilization-in-place).
Der durch die Bereitstellungsprozeduren bedingte Nutzungsausfall der Reaktoren kann insbesondere bei kurzen Nutzungsperioden und häufigem Produktwechsel in der Größenordnung der Reaktorverfügbarkeit liegen. Betroffen sind im USP der biotechnologischen Produktion z. B. die Prozessschritte der Medienherstellung und Fermentation und im DSP das Solubilisieren, Einfrieren, Auftauen, pH-Adjustieren, Produkttrennung wie z. B. durch Chromatographie, Fällen, oder Kristallisieren, das Umpuffern und die Virusinaktivierung.

Die regulatorischen Anforderungen sind dabei im Downstream-Bereich eine keimarme Prozessführung. Daher ist eine sterile Fahrweise im Falle des Batch-Betriebes nicht gefordert. In einem kontinuierlichen Verfahren jedoch wird die Reinigung des Proteins über einen längeren Zeitraum möglichst ohne Reinigungsschritte betrieben. Dies geschieht bevorzugt ohne Sterilisationsschritte während der Reinigung. Dabei ist aber das Verkeimungsrisiko um ein vielfaches höher als bei einem reinen Batchbetrieb.

WO2012/078677 beschreibt ein Verfahren und eine Anlage zur kontinuierlichen Aufbereitung von biopharmazeutischen Produkten durch Chromatographie und deren Integration in einer Produktionsanlage insbesondere in einer Einweg-Anlage. Obwohl WO2012/078677 Ansätze zur kontinuierlichen Produktion von biopharmazeutischen und biologischen Produkten liefert, reicht die offenbarte Lösung in der Praxis nicht aus. WO2012/078677 offenbart auch nicht die Verwendung einer sterilisierten Chromatographiesäule.

US 2014/0255994 A1 offenbart ein integriertes kontinuierliches Verfahren zur Herstellung von therapeutischen Proteinen. US 2014/0255994 A1 offenbart allerdings nicht das Merkmal, dass in so einem Verfahren sterilisierte Chromatographiesäulen verwendet werden könnten.

EP 2 182 990 A1 offenbart ein Verfahren zur Sterilisation von Chromatographiesäulen durch Verwendung von heißem Wasserdampf.

### Zunächst seien einige Begriffe näher definiert.

Ein kontinuierliches Verfahren meint im Rahmen der Erfindung jeden Prozess zur Durchführung von mindestens zwei Prozessschritten in Serie, in dem der Ausgangsstrom eines vorgeschalteten Schrittes in einen nachgeschalteten Schritt befördert wird. Der nachgeschaltete Schritt beginnt die Bearbeitung des Produktstroms, bevor der vorgeschaltete Schritt abgeschlossen ist. Üblicherweise wird in einem kontinuierlichen Verfahren immer ein Teil des Produktstroms in der Produktionsanlage befördert und wird als "kontinuierlicher Produktstrom" bezeichnet. Entsprechend bedeutet eine kontinuierliche Beförderung oder Transfer eines Produktstroms von einer vorgeschalteten Unit in eine nachgeschaltete Unit, dass die nachgeschaltete Unit bereits arbeitet, bevor die vorgeschaltete Unit außer Betrieb genommen wird, d.h. dass zwei nacheinander geschaltete Units den Produktstrom gleichzeitig prozessieren, der sie durchfließt.

Der Begriff "keimreduziert" meint im Rahmen der Erfindung einen Zustand reduzierter Keimzahl, also einer Zahl an Mikroorganismen pro Flächen- oder Volumeneinheit von nahezu Null, der durch eine geeignete Keimreduktionsmethode erreichbar ist, wobei diese Keimreduktionsmethode ausgewählt sein kann aus Gamma-Bestrahlung, Beta-Bestrahlung, Autoklavieren, Ethylenoxid (ETO)-Behandlung und "steam-in-place" (SIP)-Behandlung.

Der Begriff "Einwegartikel" bedeutet im Rahmen der Erfindung, dass die betreffenden produkt-berührten Teile, insbesondere Apparaturen, Behältern, Filter und Verbindungselemente, zum einmaligen Gebrauch mit anschließendem Wegwerfen geeignet sind (Englisch "disposable"), wobei diese Behälter sowohl aus Kunststoff als auch aus Metall sein können. Im Rahmen der Erfindung umfasst der Begriff auch Mehrwegartikel, etwa aus Stahl, die im erfindungsgemäßen Verfahren nur einmal verwendet werden und dann im Verfahren nicht mehr zum Einsatz kommen. Diese Mehrwegartikel, z.B. aus Stahl, werden dann im Rahmen der Erfindung auch "als Einwegartikel benutzte" Gegenstände bezeichnet. Solche eingesetzten Einwegartikel können dann im erfindungsgemäßen Verfahren auch als "disposabel" bzw. "Single-Use"- Artikel bezeichnet werden ("SU Technologie"). Dadurch wird der keimreduzierte Zustand des erfindungsgemäßen Verfahrens und modularer Anlage noch weiter verbessert.

Der Begriff "Produktstrom" meint im Rahmen der Erfindung das partikel-freie Fluid aus einer heterogenen Zellkultur-Fluid-Mischung, welche das Produkt enthält, sowie das Ergebnis jeder anderen Verfahrensschritte des erfindungsgemäßen Verfahrens, also den Produktstrom nach Filtration, nach Chromatographie, nach Virenabreicherung, nach Ultrafiltration, nach Diafiltration, oder nach weiteren Schritten des erfindungsgemäßen Verfahrens, wobei diese Produktströme dann unterschiedliche Konzentrationen und Reinheitsgrade aufweisen können.

Der Begriff "Virenabreicherung" meint im Rahmen der Erfindung eine Verringerung der Konzentration an aktiven Viren pro Volumeneinheit des zu behandelnden Fluids, bis hin zur vollständigen Inaktivierung und/oder Entfernung der in dem zu behandelnden Fluid enthaltenen Viren.

Der Begriff "Mikrobizid" meint im Rahmen der Erfindung einen Stoff, welcher das Wachstum von Mikroorganismen verlangsamen oder ganz hemmen kann, wobei dieses Mikrobizid in Form eines mikrobizid-haltigen Puffers, insbesondere im Rahmen des erfindungsgemäßen Verfahrens während einer Ultrafiltration verwendet werden kann.

Der Begriff "Bubble-Trap" meint im Rahmen der Erfindung eine Vorrichtung zum Auffangen von Gasblasen, wobei dabei das betreffende Fluid entgast wird.

Der Begriff "modular" meint im Rahmen der Erfindung, dass die einzelnen Schritte des erfindungsgemäße Verfahrens in separaten, miteinander verbundenen Modulen durchgeführt werden können, wobei die Module vorkonfiguriert und keimreduziert sind und geschlossen, in unterschiedlichen Kombinationen miteinander verschaltet werden können.

Der Begriff "modulare Anlage" meint im Rahmen der Erfindung eine Serie von miteinander verbundenen Modulen ("Units") zur Durchführung von mindestens zwei Downstream- und / oder Upstream-Schritten, in denen ein Fluid ("Produktstrom") befördert werden kann. Erfindungsgemäß sind die Units zur kontinuierlichen Durchführung eines Schrittes geeignet und können mit einem kontinuierlichen Fluidstrom ("Produktstrom") betrieben werden. Die einzelnen Module der "modularen Anlage" können dabei in jeder Kombination miteinander verschaltet werden. Beispiele für Module im Sinne der Erfindung sind das Filtrationsmodul 2, das Chromatographiemodul 3, das Ultrafiltrationsmodul 6, das Diafiltrationsmodul 7 und das Dialysemodul 8.

Der Begriff "geschlossen" bedeutet im Rahmen der Erfindung die Fahrweise des erfindungsgemäßen Verfahrens und der erfindungsgemäßen modularen Anlage, welche so gefahren werden, dass das Produkt, welches mit diesem Verfahren und dieser modularen Anlage produziert und/oder aufbereitet wird, nicht an die Raumumgebung exponiert wird. In das erfindungsgemäße geschlossene Verfahren und die entsprechende erfindungsgemäße geschlossene modulare Anlage können von außen Materialien, Gegenstände, Puffer und dergleichen zugegeben werden, wobei aber diese Zugabe in einer solchen Weise erfolgt, dass eine Exposition des produzierten und/oder aufbereiteten Produktes an die Raumumgebung vermieden wird.

Die im Stand der Technik bekannten, üblichen Verfahren weisen eine Reihe von Nachteilen auf, welche im Folgenden behandelt werden.

Bekannte Verfahren zur Herstellung von biopharmazeutischen und biologischen Produkten umfassen üblicherweise folgende Produktionsschritte, die miteinander verschaltet werden:
1. Perfusionskultur
2. Zellrückhaltesystem,
alternativ zu Schritt 1 und 2 kann auch eine Feedbatchkultur vorgesehen sein,
3. Zellabtrennung
4. Puffer- bzw. Medienaustausch bevorzugt mit Konzentrierung
5. BioBurden-Reduzierung bevorzugt durch Sterilfiltration
6. Capture Chromatographie

Üblicherweise werden weitere Schritte zur weiteren Reinigung des Produktstroms durchgeführt, insbesondere:
7. Virusinaktivierung
8. Neutralisation, und
9. Optional eine weitere Tiefenfiltration, BioBurden-Reduzierung (Sterilfiltration).

Angesichts der hohen Qualitätsstandards bei der Herstellung von Biopharmazeutika folgen üblicherweise darüber hinaus folgende Schritte:
10. Chromatographische Zwischen- und Feinreinigung
11. BioBurden Reduzierung z. B. Sterilfiltration
12. Virenfiltration
13. Pufferaustausch und bevorzugt Konzentrierung, und
14. Sterilfiltration.

Bei der oben beschriebenen Herstellung stellen Zellen in einem Fermenter mit Nährstofflösung ein biologisches Produkt her, etwa ein Protein, beispielsweise ein therapeutisches Protein. Die Nährstofflösung ist dabei auch ein ideales Wachstumsmedium für Mikroorganismen, wie Bakterien und Sporen, woraus sich ein Problem hinsichtlich des ungewünschten Wachstums solcher Mikroorganismen ergibt. Dieses ungewünschte Wachstum von Mikroorganismen wird besonders bei längeren Laufzeiten ein Problem, weil die Nährstofflösung mit ansteigender Laufzeit des Verfahrens mehr und mehr kontaminiert wird, bis hin zu einem exponentiellen Wachstum von Mikroorganismen und damit Totalverlust der betreffenden Charge an hergestelltem biologischen Produkt.

Um der Forderung an ein schnelles und flexibles Neubeschicken der Produktionsanlage unter Wahrung maximaler Sauberkeit und Keimfreiheit gerecht zu werden, erfreuen sich auf dem Markt Konzepte für eine kontinuierliche Produktion bevorzugt mit Einwegtechnologie eines ständig wachsenden Interesses.

Für längere Laufzeiten eines solchen Verfahrens von mehreren Stunden über Tage bis Wochen reichen aber gewöhnliche Maßnahmen zur Sanitisierung nicht, etwa die üblichen "Clean-in-Place" (CIP)-Maßnahmen, wie z.B. Sanitisierung durch 1M NaOH. Bei Laufzeiten ab mehreren Stunden haben solche gewöhnlichen Verfahren und Anlagen daher den Nachteil, dass sie hinsichtlich möglicher Kontamination und/oder möglichen Keimwachstums sehr anfällig sind.

Es bestand daher ein Bedarf an einem Verfahren zur kontinuierlichen Reinigung eines Produktes aus einer heterogenen Zellkultur-Fluid-Mischung, welches durch seine weitgehende Keimarmut eine kontinuierliche Fahrweise von bis zu mehreren Wochen ermöglicht.

Es war daher die Aufgabe der vorliegenden Erfindung, ein Verfahren und eine entsprechende Anlage zu entwickeln, mit dem ein Produkt, etwa ein Protein, kontinuierlich über einen Zeitraum von mehreren Stunden bis hin zu mehreren Wochen gereinigt werden kann.

Die Erfindung löst diese Aufgabe durch Bereitstellen eines Verfahrens zur kontinuierlichen, keimreduzierten Produktion und/oder Aufbereitung eines biopharmazeutischen, biologischen makromolekularen Produktes aus einer heterogenen Zellkultur-Fluid-Mischung, umfassend die Schritte:
(a) Bereitstellen eines partikel-freien Fluids aus einer heterogenen Zellkultur-Fluid-Mischung, welche das Produkt enthält, in Form eines Produktstroms,
(b) mindestens eine Filtration, wobei ein Filtrat erhalten wird,
(c) wenigstens zwei Chromatographieschritte zur Reinigung des Produktes,
(d) mindestens eine Virenabreicherung,
(e) mindestens eine Ultrafiltration und/oder mindestens eine Diafiltration des Produktstroms der Schritte (b), (c), und/oder (d),
dadurch gekennzeichnet, dass die wenigstens zwei Chromatographieschritte aus (c) eine Reinigung über wenigstens jeweils zwei Chromatographiesäulen und/oder Membranadsorber umfasst und, dass das Verfahren geschlossen und modular durchgeführt wird.

Die Grundprinzipien des erfindungsgemäßen Verfahrens beruhen auf seinen vier Kernprinzipien und damit Kernmerkmalen:
1. kontinuierliche
2. keimreduzierte
3. geschlossene, und
4. modulare Produktion eines biopharmazeutischen, biologischen makromolekularen Produktes.
Diese vier Merkmale gemeinsam verringern das gewöhnlich auftretende Problem des ungewünschten Wachstums von Mikroorganismen derart drastisch, dass Laufzeiten des erfindungsgemäßen Verfahrens in einer kontinuierlichen Fahrweise von bis zu 8 Wochen möglich werden.

Das in Schritt a) bereitgestellte partikel-freie Fluid aus einer heterogenen Zellkultur-Fluid-Mischung kann bevorzugt aus einem kontinuierlichen Perfusions- und Fermentationsprozesses stammen, etwa eine Zellkultur bzw. Gewebekultur, oder ein Perfusionsreaktor. Dabei können sogar mehr als ein Perfusionsreaktor parallel betrieben werden, etwa zwei Perfusionsreaktoren.

Das Fluid kann zunächst durch geeignete Zellrückhaltesysteme, etwa einen Schrägplattenabscheider (Settler) kontinuierlich abgeführt werden, durch den ein Großteil der Zellen zurückgehalten werden kann. Durch nachfolgende Filtrations- und/oder Zentrifugationsschritte oder andere geeignete Trennverfahren kann dann das Fluid von im Fluid enthaltenden Partikeln befreit werden, wodurch ein partikel-freies Fluid erhalten wird, in welchem sich das biopharmazeutische, biologische makromolekulare Produkt befindet.

Der Filtrationsschritt (b) kann z.B. eine Filtration des nach Schritt (a) erhaltenen partikel-freien Fluids sein, wobei ein Filtrat erhalten wird. Das erfindungsgemäße Verfahren kann aber auch weitere Filtrationsschritte an geeigneten Stellen des Verfahrens umfassen.
Der Filtrationsschritt b) kann durch geeignete Filterverfahren erfolgen, z.B. einen 0,2µm Filter, oder mehrerer parallel betriebene Filter. Ein geeigneter Filter für den Filtrationsschritt ist beispielsweise ein Sartoguard NF 0,2µm Filter, von dem mehrere parallel betrieben werden können.
In einer weiteren Ausführungsform des erfindungsgemäßen Verfahrens umfasst der Filtrationsschritt (b) einen Tiefenfilter mit einer zusätzlichen Möglichkeit der Abreicherung von Kontaminanten wie DNA, ProtA, HCP. Dies können Tiefenfilter mit ausreichendem Zetapotential (Zetapor 3M, Posidyne, Pall) oder Tiefenfilter mit Aktivkohle (Millistak MerckMillipore) sein.

Die wenigstens zwei Chromatographieschritte zur Reinigung des Produktes des Schrittes c) umfassen eine Reinigung über wenigstens jeweils zwei Chromatographiesäulen und/oder Membranadsorber. Dabei können die Chromatographiesäulen und/oder Membranadsorber jedes geeignete Bindungsprinzip aufweisen, etwa Affiniät des Produktes zu einem Liganden, ionische Wechselwirkungen, Metalchelat-Bindung, hydrophobe Interaktionen oder van-der-Waals-Kräfte. Beispielsweise kann der erster Chromatographieschritt der wenigstens zwei Chromatographieschritte eine Affinitätschromatographie sein (z.B. ein Ligand mit Affinität zum Produkt, wie z.B. Protein A, Protein G, Protein L, IgM, IgG und ein von Protein A, Protein G und Protein L, IgM, IgG unterschiedlichen rekombinanten Protein, welches eine Affinität für das Produkt hat). Danach folgt dann ein weiterer (zweiter) Chromatographieschritt, etwa eine Chromatographie über ionische Wechselwirkungen.

Das erfindungsgemäße Verfahren ist hier flexibel und kann in Schritt c) je nach zu erzielendem Reinheitsgrad und Konzentration des Produktes jedes geeignete Chromatographieprinzip in jeder Reihenfolge umfassen.

Der technische Effekt der Verwendung von wenigstens zwei Chromatographieschritten über wenigstens jeweils zwei Chromatographiesäulen und/oder Membranadsorbern gemäß Schritt c), besteht darin, dass in der Regel ein einzelner Chromatographieschritt keine ausreichende Aufreinigung der Kontaminaten wie z.B. Host Cell Impurities, Aggregate, DNA, Protein A usw. gewährleisten kann.
Außerdem wird dadurch eine kontinuierliche Produktion in einem Chromatographieschritt ermöglicht, da mindestens eine Chromatographiesäule und/oder Membranadsorber mit ungereinigtem Produkt beladen werden kann, während mindestens eine andere Chromatographiesäule und/oder Membranadsorber regeneriert bzw. eluiert werden kann, wodurch eine kontinuierliche und effektive Fahrweise des Verfahrens erzielt werden kann.

In einer weiteren Ausführungsform des erfindungsgemäßen Verfahrens erfolgen zwischen den wenigstens zwei Chromatographieschritten in (c) und/oder nach der Vireninaktivierung in Schritt (d) ein oder mehrere weitere Schritte zur Einstellung des pH-Wertes und/oder zur Einstellung der Leitfähigkeit und/oder Filtrations- und/oder Konzentrationsschritte und/oder ein Pufferaustausch. Dadurch wird eine an die Bedingungen anpassbare Fahrweise des Prozesses ermöglicht.

Die mindestens eine Virenabreicherung des Schrittes d) kann insbesondere durch Einstellen des pH-Werts des partikel-freien Fluides erfolgen, bevorzugt auf einen pH-Wert von ≤ 4,0. Einstellen des pH-Wertes des zu inaktivierenden partikel-freien Fluids auf ≤ 4,0 kann beispielsweise durch Zugabe von HCl-Lösung erfolgen. Die Zugabe erfolgt typischerweise im Vorfeld der Vorrichtung zur Virusabreicherung. Üblicherweise wird der pH-Wert auf >4 mit einer Base beispielsweise Natriumhydroxid-Lösung (NaOH) eingestellt, um die Virusabreicherung zu beenden.

Die mindestens eine Virenabreicherung des Schrittes d) kann aber auch durch eine solventdetergent Schritt erfolgen, bei dem eine Virenabreicherung durch Lösungsmittel- / Detergenz erzielt wird.

In einer weiteren Ausführungsform kann die Virenabreicherung auch durch UV-Behandlung und/oder durch thermische Behandlung erzielt werden.
Die mindestens eine Virenabreicherung des Schrittes d) kann insbesondere in einer Verweilstrecke erfolgen, in die ein segmentierter Produktstrom eingebracht werden kann.

In einer weiteren Ausführungsform des erfindungsgemäßen Verfahrens sind alle in den Schritten (a) bis (e) eingesetzten, produkt-berührten Elemente durch eine geeignete Keimreduktionsmethode keimreduziert.
Bevorzugt kann die Keimreduktionsmethode ausgewählt sein aus der Gruppe bestehend aus Gamma-Bestrahlung, Beta-Bestrahlung, Autoklavieren, Ethylenoxid (ETO)-Behandlung, Ozon-Behandlung (O₃), Wasserstoffperoxid-Behandlung (H₂O₂) und Steam-in-place (SIP) Behandlung.

Entsprechend sind bevorzugt auch die in dem erfindungsgemäßen Verfahren eingesetzten Gegenstände und Elemente der Module, die mit Produktstrom in Kontakt treten, keimreduzierbar und/oder sterilisierbar, bevorzugt autoklavierbar, gamma-bestrahlbar, mit Ethylenoxid (ETO) begasbar, mit Ozon (O₃) behandelbar, mit Wasserstoffperoxid (H₂O₂) behandelbar oder mit einer Steam-in-place (SIP)-Behandlung behandelbar, was einen keimreduzierten oder sogar aseptischen Betrieb des erfindungsgemäßen Verfahrens ermöglicht.

In einer weiteren Ausführungsform des erfindungsgemäßen Verfahrens sind alle ab Filtrationsschritt (b) verwendeten produktberührten Elemente Einwegartikel oder werden als Einwegartikel benutzt. Solche eingesetzten Einwegartikel können dann im erfindungsgemäßen Verfahren auch als "disposabel" bzw. "Single-Use"-Artikel bezeichnet werden ("SU Technologie"). Dadurch wird der keimreduzierte Zustand des Verfahrens verbessert.

In einer weiteren Ausführungsform des erfindungsgemäßen Verfahrens werden alle Eingangsfluide durch einen Keimreduktionsfilter filtriert, wie etwa einen Sartoguard NF Filter der Firma Sartorius.

Dabei können bevorzugt alle Ausgänge durch eine Keimbarriere geschützt sein, die ein Rückwachstum von Mikroorganismen verhindern. Beispielsweise kann auch hier ein Sartoguard NF Filter der Firma Sartorius als Keimbarriere verwendet werden. Eine zusätzliche Sicherheit der Keimbarriere 11 kann durch eine Wechselschaltung von Filtern und/oder Abfallleitung erzielt werden. Eine weitere Maßnahme zur Gewährleistung der keimreduzierten Bedingungen kann durch eine periodische Sanitisierung der Abfallleitung vorzugsweise nach der Filtration mit z.B. NaOH Lösung erreicht werden. Weitere Verfahren wären UV Bestrahlung und Hitzebehandlung.

Die modularen Verfahrensschritte des erfindungsgemäßen Verfahrens werden in einer weiteren Ausführungsform bevorzugt in Modulen durchgeführt, wobei die Module miteinander verbunden sind.
Dabei können bevorzugt die Module durch Verschweißen oder durch aseptische Konnektoren miteinander verbunden sein. Zum Verschweißen der Module kann z.B. das Gerät "TC Welder" der Firma Sartorius verwendet werden.

In einer weiteren Ausführungsform des erfindungsgemäßen Verfahrens sind in den Schritten (a) bis (e) alle verwendeten Flüssigkeiten, Gase und Feststoffe keimreduziert. Dabei erfolgt die Keimreduktion bevorzugt durch eine Filtration durch einen Filter mit einer Porengröße von bevorzugt ≤ 0,45 µm. Dabei wird bevorzugt während des Verfahrens keine Inprocess-Sterilisierung durchgeführt. In anderen Ausführungsformen kann auch die Keimreduktion durch eine Filtration durch einen Filter mit einer Porengröße von bevorzugt ≤ 0,20 µm durchgeführt werden.

In einer weiteren bevorzugten Ausführungsform des erfindungsgemäßen Verfahrens wird vor dem Chromatographie-Schritt (c) ein Entgasen aller Fluide durchgeführt, welche auf die wenigstens zwei Chromatographiesäulen kommen, wobei das Entgasen vorzugsweise durch mindestens eine Bubble-Trap und/oder durch mindestens eine hydrophobe Mikrofiltrationsmembran über Vakuum und/oder durch Behandeln mit Ultraschall und/oder durch Durchperlen mit einem schlecht löslichen Gas, wie z.B. Helium erfolgt.

Dabei ist die Nutzung einer hydrophoben Mikrofiltrationsmembran über Vakuum für die Einhaltung der Keimfreiheit in der kontinuierlichen Führung des erfindungsgemäßen Verfahrens und der erfindungsgemäßen Anlage bevorzugt, da diese sich als besonders vorteilhaft im Vergleich zur Bubble-Trap erwiesen hat. Die verwendete hydrophobe Mikrofiltrationsmembran kann insbesondere ein MicroModule der Firma Membrana sein.

In einer besonders bevorzugten Ausführungsform des erfindungsgemäßen Verfahrens wird das partikel-freie Fluid aus Schritt a) mindestens einer Ultrafiltration gegen einen mikrobizid-haltigen Puffer unterzogen. Durch die Ultrafiltration werden die in dem Fluid enthaltenden Nährstoffe gegen einen mikrobizid-haltigen Puffer ausgetauscht, wodurch Mikroorganismen / Keimen die Wachstumsgrundlage in dem Fluid entzogen werden soll. Dadurch wird die Keimreduktion des Verfahrens zusätzlich verbessert.

Das dabei verwendete Mikrobizid, oder ein oder mehrere Mikrobizide können bevorzugt ausgewählt sein aus der Gruppe bestehend aus Imidazol, Benzoesäure, Sorbinsäure, para-Hydroxybenzoesäure-Ester, Sulfite, Disulfite, Azide, Orthophenylphenol, Nisin, Natamycin, Hexamethylentetramin, Dimethyldicarbonat, Nitrite, Nitrate, Essigsäure, Ascorbinsäure, Isoascorbinsäure, L-Milchsäure, Propionsäure, Borsäure und Lysozym.

Die in dem mikrobizid-haltigen Puffer enthalten Mikrobizide können weiterhin sein ein oder mehrere Mikrobizide aus der Gruppe bestehend aus:
E210 bis E213 Benzoesäure und ihre Salze, 0,05-0,1% in Lösungsmittel in saurem Milieu,
2-3g/kg in LM;
E200 bis E203: Sorbinsäure und ihre Salze, 300-2000 mg/kg;
E214 bis E219: PHB Ester (para-Hydroxybenzoesäure-Ester, Parabene), Butyl- und Propylparaben
E220 bis E228: Sulfite und Disulfite,
E231 und E232: Orthophenylphenol, 12 mg/kg;
E234: Nisin,
E235: Natamycin,
E239: Hexamethylentetramin, 25mg/kg;
E242: Dimethyldicarbonat;
E249-E252: Nitrite und Nitrate, 300 mg/kg;
E260: Essigsäure, 0,5-3%;
E300-E302: Ascorbinsäure, 300 mg/kg;
E315-E316: Isoascorbinsäure, 1500 mg/kg;
E261-E263: Azetat;
E270 L-Milchsäure;
E280 bis E283: Propionsäure und ihre Salze, 1-3 g/kg;
E284 und E285: Borsäure, max. 4g/kg;
E1105 Lysozym; und
Azide.

In einer bevorzugten Ausführungsform des erfindungsgemäßen Verfahrens ist das biopharmazeutische, biologischen makromolekulare Produkt ein Protein oder Peptid, dass ausgewählt ist aus der Gruppe bestehend aus monoklonalen Antikörpern, polyklonalen Antikörpern, rekombinanten Proteinen und Impfstoffen, bevorzugt DNA- und RNA-Impfstoffen.

Die in Schritt c) verwendeten die Chromatographiesäulen und/oder Membranadsorber können jedes geeignete Bindungsprinzip aufweisen, etwa Affiniät des Produktes zu einem Liganden, ionische Wechselwirkungen, Metalchelat-Bindung, hydrophobe Interaktionen oder reine van-der-Waals-Kräfte. Beispielsweise kann der erster Chromatographieschritt der wenigstens zwei Chromatographieschritte eine Affinitätschromatographie sein (z.B. ein Ligand mit Affinität zum Produkt, wie z.B. Protein A, Protein G, Protein L, IgM, IgG und ein von Protein A, Protein G und Protein L, IgM, IgG unterschiedlichen rekombinanten Protein, welches eine Affinität für das Produkt hat). Danach folgt dann ein weiterer (zweiter) Chromatographieschritt, etwa eine Chromatographie über ionische Wechselwirkungen.
Das erfindungsgemäße Verfahren ist hier flexibel und kann in Schritt c) je nach zu erzielendem Reinheitsgrad und Konzentration des Produktes jedes geeignete Chromatographieprinzip in jeder Reihenfolge umfassen.
In einer besonders bevorzugten Ausführungsform des erfindungsgemäßen Verfahrens umfassen die wenigstens zwei Chromatographiesäulen und/oder Membranadsorber des Schrittes (c) einen Ligand, der bevorzugt ausgewählt ist aus der Gruppe bestehend aus Protein A, Protein G, Protein L, IgM, IgG und einem von Protein A, Protein G und Protein L, IgM, und IgG unterschiedlichen rekombinanten Protein, welches eine Affinität für das Produkt hat.

In einer bevorzugten Ausführungsform des erfindungsgemäßen Verfahrens weist das Verfahren der Schritte (a) bis (e) eine Laufzeit von mindestens 4 Stunden auf, bevorzugt von mindestens 8 Stunden, bevorzugt von mindestens 12 Stunden, bevorzugt von mindestens 24 Stunden, weiterhin bevorzugt von mindestens 48 Stunden, weiterhin bevorzugt von mindestens 7 Tagen, weiterhin bevorzugt von mindestens 4 Wochen, und besonders bevorzugt von mindestens 8 Wochen auf. Eine derart lange Laufzeit von mehreren Wochen kontinuierlicher Fahrweise wird nur ermöglicht durch die geschlossene, modulare und vor allem keimreduzierte Fahrweise des Prozesses.

In einer bevorzugten Ausführungsform des erfindungsgemäßen Verfahrens wird zwischen den Schritten a) bis e) und/oder danach mindestens ein Filtrationsschritt umfassend mindestens einen Filter durchgeführt.

In einer besonders bevorzugten Ausführungsform des erfindungsgemäßen Verfahrens wird der Filter automatisch unter keimreduzierten Bedingungen gewechselt, wobei bevorzugt der automatische Filterwechsel die folgenden Schritte umfasst:
(i) Umschalten des Flussweges auf einen neuen Filter bei Überschreiten eines Schwellenwertes am Drucksensor auf der Unfiltratseite unter Verschließen des Flussweges, wobei das Produkt im verbrauchten Filter bevorzugt durch ein Gas oder eine Flüssigkeit in die Filtratseite gedrückt wird,
   oder bei Überschreiten einer maximalen Zeit des verbrauchten Filters im Flussweg, oder bei Überschreiten eines maximalen Volumens an Filtrat durch den verbrauchten Filter,
(ii) Entlüften des neuen Filters über eine Luftfilter mit einer Porengröße von bevorzugt <= 0,25 µm am Entlüftungsventil des neuen Filters, bevorzugt unter Beförderung von Produkt in den neuen Filter durch eine Feedpumpe, oder in einen keimreduziert angeschlossenen, geschlossenen Bag,
(iii) Detektion der Beendigung der Entlüftung des neuen Filters auf der Unfiltratseite durch den Drucksensor oder einen Füllstandssensor oder eine Waage oder einen Flüssigkeitsdetektor,
(iv) Öffnen des Filtratausgangs und Verschluss des Flussweges zwischen Entlüftungsventil und Luftfilter durch ein Ventil, und
(v) Austausch des alten Filters gegen einen neuen Filter.

Die gleichzeitige oder nachgeschaltete Förderung von Produkt in den neuen Filter kann z.B. durch eine Feedpumpe erfolgen.

Das bedeutet, dass in einer weiteren Ausführungsform des erfindungsgemäßen Verfahrens, der Filterwechsel (Umschalten von einem verbrauchten auf ein neues Filterelement) automatisch erfolgen kann. Als problematisch erweist sich hierbei die Filterentlüftung, die aber erforderlich ist, und die bei den zur Zeit erhältlichen Filtern manuell durchgeführt werden muss. Zunächst wird der Filter einer Keimreduktionsmethode durch ggf. ETO. Autoklavieren oder Gammabestrahlung unterworfen und dann mit dem Prozess verbunden. Danach kann der Filter auf der Unfiltratseite befüllt werden, während das Entlüftungsventil offen ist, welches nach dem erfolgreichen Entlüften geschlossen werden muss, damit die eigentliche Filtration durchgeführt werden kann. In einem Batch-Prozess ist eine stark keimreduzierte Handhabung auf der Unfiltratseite nicht erforderlich, da es in den kurzen Zeiträumen nur auf möglichst keimfreies Filtrat ankommt. In einem kontinuierlichen Prozess ist jedoch auch die möglichst keimfreie Führung der Unfiltratseite erforderlich, um eine Verkeimung des Prozesses zu verhindern. Im Stand der Technik muss das Entlüftungsventil durch eine Drehbewegung nach dem Befüllen des Filters manuell geschlossen werden, damit die eigentliche Filtration durchgeführt werden kann. Diese Drehbewegung ist nur sehr aufwendig zu automatisieren, da dabei auch noch eine axiale Verschiebung des Drehkörpers notwendig ist. Durch das axiale Verschieben des Drehkörpers samt der darauf angebrachten Dichtungen wird die Grenze verschoben. In Kombination mit einer vorher durchgeführten Keimreduktion ergibt sich das Problem, dass beim Keimreduzieren mit geschlossenem Entlüftungsventil die Keimgrenze bei Öffnen des Entlüftungsventils verschoben wird. Dabei wird ein keimbehafteter Bereich in den keimreduzierten Bereich eingebracht, und die Keimreduktion damit aufgehoben. Ein Keimreduzieren mit geöffnetem Filterventil ist nicht zu empfehlen, da die offene Position keine feste Position hat, und es dadurch leicht zu einer Beschädigung des Ventils kommen kann. Außerdem ist die offene Position meist wackelig, so dass es durch das normale Handling des Filters zu einer Verschiebung der Keimgrenze kommen kann.

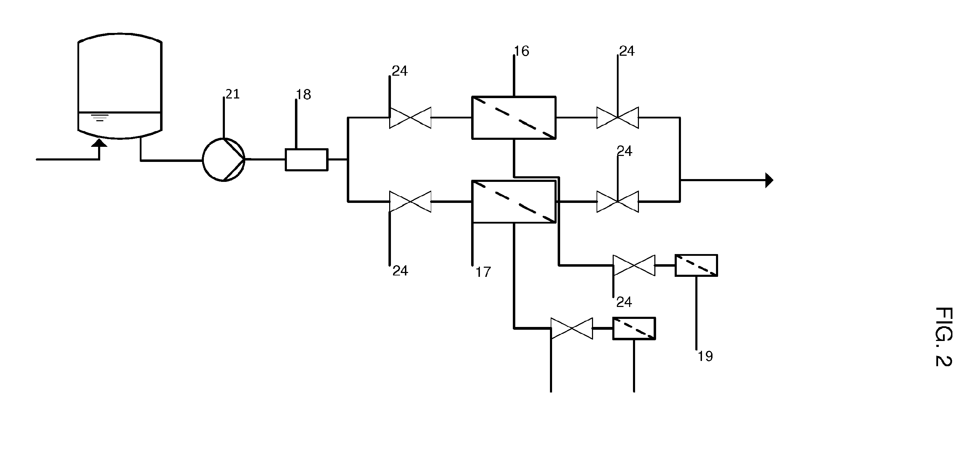

Für die automatische Filterentlüftung ist es vorteilhaft, die Drehbewegung des Entlüftungsventils während der Inbetriebnahme zu vermieden, damit die Entlüftung nur durch einfache Maßnahmen durchgeführt werden kann. Das Entlüftungsventil kann nun so modifiziert werden, dass es auch im geschlossenen Zustand durchgängig ist, jedoch weiterhin zuverlässig zur Umwelt hin abdichtet. Das Ventil befindet sich dadurch im sicheren Zustand "geschlossen", was durch einen festen Sitz charakterisiert ist. Der Zustand "offen" ist meist nicht eindeutig definiert, da der Ventilkörper in der Führung stark wackelt und ein Verschieben der Grenze ermöglicht. An der Tülle des Drehkörpers wird ein Schlauch angebracht, der in einem hydrophoben <=0,2 µm Luftfilter endet. Diese Anordnung wird nun vorzugsweise durch ETO, Gammabestrahlung, Autoklavieren, oder Ozon (O₃)-Behandlung oder durch Wasserstoffperoxid (H₂O₂)-Behandlung keimreduziert. Somit ist die gesamte Unfiltratseite bis zum Luftfilter am Entlüftungsventil keimreduziert bzw. keimarm. Zwischen Entlüftungsventil und Luftfilter wird der Schlauch in ein Schlauchquetschventil eingelegt, welches den Schlauch zuverlässig zu quetschen vermag. Die Entlüftung kann so vollautomatisch und keimarmst erfolgen. Der Filter wird befüllt, bis Flüssigkeit durch das Entlüftungsventil und den Schlauch in den Luftfilter eintritt. Der hydrophobe Entlüftungsfilter blockiert die Flüssigkeit. Gleichzeitig blockiert die Prozessanlage durch ein Ventil auf der Filtratseite den Produktionsstrom, so dass es zu einem Druckanstieg vor dem Filter kommt. Dieser wird über einen geeigneten Sensor detektiert. Ist ein bestimmter Schwellenwert des Drucks überschritten, wird das Quetschventil des Entlüftungsschlauches geschlossen, und das Ventil auf der Filtratseite geöffnet. In dieser Vorgehensweise kann mit einem modifizierten Entlüftungsventil der Filter automatisch ohne manuellen Eingriff modifiziert werden.
Dieser automatische Filterwechsel ist in **Figur 2** beispielhaft gezeigt, wo schematisch das Arbeitsprinzip des Filtrationsschrittes des Verfahrens und der Anlage unter Ersetzen eines verbrauchten Filters 17 durch Einschweißen eines neuen Filters 16 veranschaulicht wird.

Das fertig gereinigte biopharmazeutischen, biologischen makromolekularen Produkte aus der heterogenen Zellkultur-Fluid-Mischung kann am Schluss des Verfahrens durch eine finale Filtration, bevorzugt durch einen Filter mit einer Porengröße von 0,2µm ein letztes Mal gefiltert werden. Die finale Filtration kann beispielsweise über gammabestrahlte Sartopore 2 Kapsulen (Midicap size 7, 0,05m²) in ein gammabestrahltes 5L GE ReadCircuit Bag erfolgen. Wenn der Füllstand des finalen Bags ausreichend ist, dann kann dieses Bag abgeschweißt werden und ein neues Bag an den Prozess angeschweißt werden.

Die Erfindung löst diese Aufgabe weiterhin durch Bereitstellen einer modularen Anlage zur kontinuierlichen, keimreduzierten Produktion und/oder Aufbereitung eines biopharmazeutischen, biologischen makromolekularen Produktes aus einer heterogenen Zellkultur-Fluid-Mischung, umfassend folgende Module:
(a) mindestens ein Filtrationsmodul,
(b) mindestens ein Chromatographiemodul, umfassend mindestens jeweils zwei Chromatographiesäulen und/oder Membranadsorbern,
(c) mindestens ein Ultrafiltrationsmodul und/oder mindestens ein Diafiltrationsmodul und/oder mindestens ein Dialysemodul, und
(d) mindestens ein Modul zur kontinuierlichen Virenabreicherung,
   dadurch gekennzeichnet, dass die modulare Anlage geschlossen und keimreduziert ist.

Das mindestens eine Chromatographiemodul, kann aber auch mehr als zwei Chromatographiesäulen und/oder Membranadsorbern umfassen, z.B. drei oder vier Chromatographiesäulen und/oder Membranadsorber.

In einer weiteren Ausführungsform der erfindungsgemäßen modularen Anlage sind alle in den Modulen (a) bis (d) eingesetzten und mit produktberührten Elemente durch eine Keimreduktionsmethode keimreduziert, wobei die Keimreduktionsmethode bevorzugt ausgewählt ist aus der Gruppe bestehend aus Gamma-Bestrahlung, Beta-Bestrahlung, Autoklavieren, Ethylenoxid (ETO)-Behandlung, Ozon-Behandlung (O₃), Wasserstoffperoxid-Behandlung (H₂O₂) und steam-in-place (SIP) Behandlung.

Bevorzugt sind die modular Anlage selber, sowie die Elemente der Module, die mit Produktstrom in Kontakt treten, keimreduzierbar und/oder sterilisierbar, bevorzugt autoklavierbar, gamma-bestrahlbar, mit Ethylenoxid (ETO) begasbar, mit Ozon (O₃) behandelbar, mit Wasserstoffperoxid (H₂O₂) behandelbar oder mit einer Steam-in-place (SIP)-Behandlung behandelbar, was einen keimarmen oder sogar aseptischen Betrieb der erfindungsgemäßen modularen Anlage ermöglicht.

In einer weiteren Ausführungsform der erfindungsgemäßen modularen Anlage sind alle in den Modulen (a) bis (d) verwendeten produktberührten Gegenstände Einwegartikel oder werden als Einwegartikel benutzt. Dabei sind die Module bevorzugt durch Verschweißen oder durch aseptische Konnektoren miteinander verbunden. Beispielsweise sind aseptische "ReadyMate"-Konnektoren der Firma GE bevorzugt in der erfindungsgemäßen modularen Anlage verwendete aseptische Konnektoren.
Bevorzugt werden fertig einsetzbare Einwegartikel ("Disposables", "ready-to-use") als gammabestrahlte Elemente verwendet.

In einer bevorzugten Ausführungsform der erfindungsgemäßen modularen Anlage gehen alle Eingangsfluide durch einen Keimreduktionsfilter, wobei bevorzugt alle Ausgänge durch eine Keimbarriere geschützt sind, die ein Rückwachstum verhindern.

Das fertig gereinigte biopharmazeutischen, biologischen makromolekularen Produkte aus der heterogenen Zellkultur-Fluid-Mischung kann am Ende der modularen Anlage durch eine finale Filtration, bevorzugt durch einen Filter mit einer Porengröße von 0,2µm ein letztes Mal gefiltert werden. Die finale Filtration kann beispielsweise über gammabestrahlte Sartopore 2 Kapsulen (Midicap size 7, 0,05m²) in ein gammabestrahltes 5L GE ReadCircuit Bag erfolgen. Wenn der Füllstand des finalen Bags ausreichend ist, dann kann dieses Bag abgeschweißt werden und ein neues Bag an den Prozess angeschweißt werden.

Die vorliegende Erfindung einschließlich bevorzugter Ausführungsformen wird in Verbindung mit den nachfolgenden Zeichnungen und dem Beispiel erläutert, ohne hierauf beschränkt zu sein. Die Ausführungsformen können beliebig miteinander kombiniert werden, sofern sich nicht eindeutig das Gegenteil aus dem Kontext ergibt.

Es zeigen:

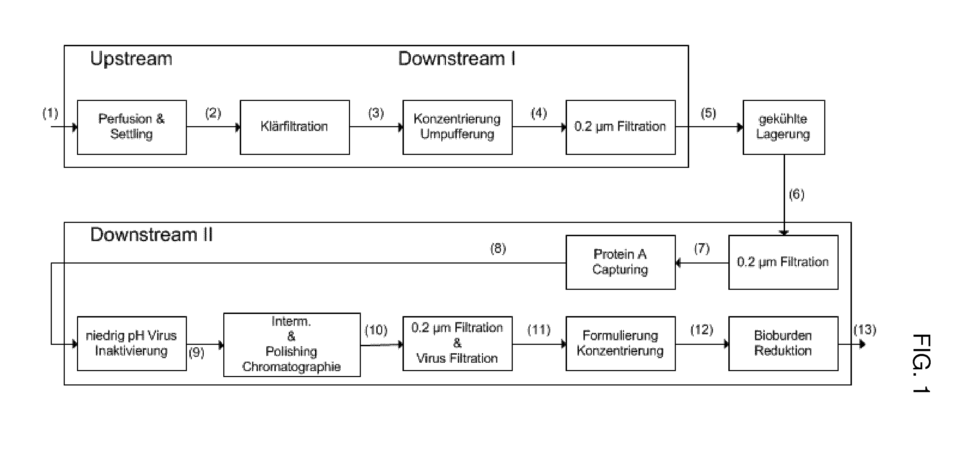

**FIG. 1** zeigt schematisch ein Verfahrensschema einer Ausführungsform des erfindungsgemäßen Verfahrens. Die Zahlen in Klammern sind Verweise auf die Massenbilanz, wie in **Beispiel 1 und Tabelle 1** aufgeführt.
**Fig. 2** zeigt schematisch das Arbeitsprinzip des Filtrationsschrittes des Verfahrens und der Anlage unter Ersetzen eines verbrauchten Filters 17 durch Einschweißen eines neuen Filters 16.

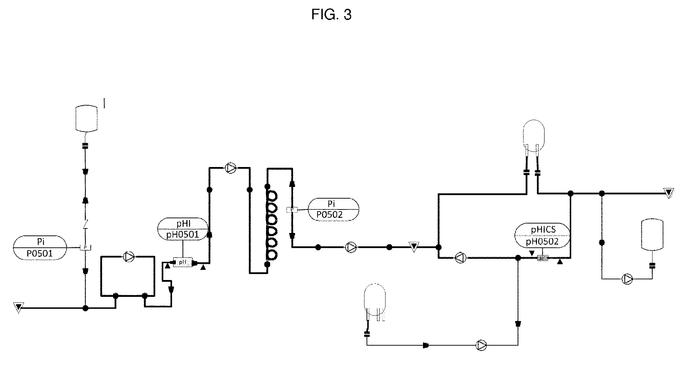

**Fig. 3** zeigt exemplarisch die zwei Verfahrensschritte Vireninaktivierung und Neutralisierung - zwei Units die modular aufgebaut sind, wobei die pH Sonden pH0501 und pH0502 autoklaviert werden und der Rest jeweils gammabestrahlt wird. Die pH Sonden pH0501 und pH0502 werden dann in ein Assembly eingeschweißt. Die Bags sind über aseptische GE ReadyMate^{®} Konnektoren angeschlossen. Die Verbindung zu Prot-A und der Filtrationseinheit ist zunächst zugeschweißt und wird dann an die jeweiligen Einheiten angeschweißt.

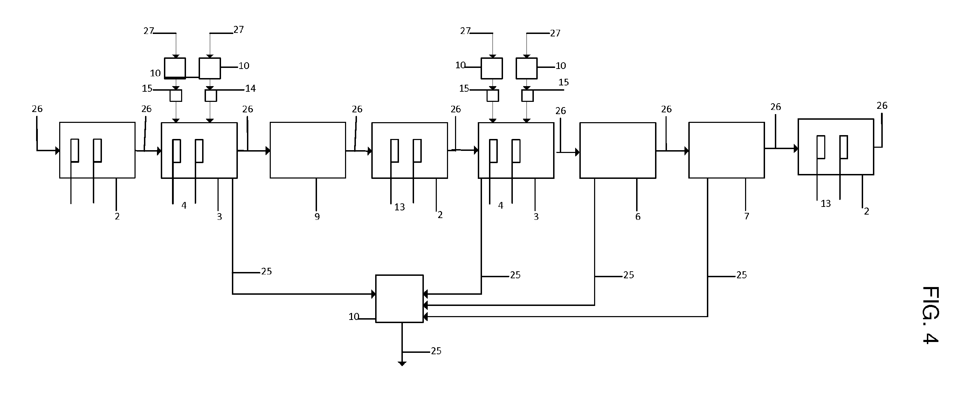

**Fig. 4** zeigt exemplarisch den modularen Aufbau der Anlage, wobei alle Eingangsströme und Ausgangsströme über eine Keimbarriere 10, 13 mit der Umgebung verbunden sind. Die exemplarische modulare Anlage 1 besteht aus drei Filtrationsmodulen 2 mit jeweils zwei wechselseitig betriebenen Filtern 13, zwei Chromatographiemodule 3 mit zwei Chromatographiesäulen 4 bzw. zwei Membranadsorber 5 ein Virenabreicherungschritt z.B. Vireninaktivierung 9, einem Ultrafiltrationsmodul 6 und einem Diafiltrationsmodul 7. Die Puffer werden über einen hydrophoben Filter 15 oder Bubble-trap 14 von Gasblasen befreit.

**Tabelle 1** zeigt die gemittelten Flussraten und Antikörper-Konzentrationen der in **Figur 1** dargestellten Positionen.

Die verwendeten Bezugsziffern sind:
1 = Modulare Anlage
2 = Filtrationsmodul
3 = Chromatographiemodul
4 = Chromatographiesäule
5 = Membranadsorber
6 = Ultrafiltrationsmodul
7 = Diafiltrationsmodul
8 = Dialysemodul
9 = Virenabreicherung
10 = Keimreduktionsfilter
11 = Keimbarriere
12 = aseptischer Konnektor
13 = Filter mit einer Porengröße von bevorzugt ≤ 0,45 µm
14 = Bubble-Trap
15= hydrophobe Mikrofiltrationsmembran
16 = neuer Filter
17 = verbrauchter Filter
18 = Drucksensor
19 = Luftfilter, Porengröße bevorzugt <= 0,25 µm
20 = Entlüftungsventil des neuen Filters 16,
21 = Feedpumpe
22 = Füllstandsensor
23 = Waage
24 = Ventil
25 = Abfallstrom
26 = Produktstrom
27 = Puffer
28 = Flüssigkeitsdetektor
29 = Bag

### Beispiel 1

Um ein Protein kontinuierlich und keimreduziert aus einer heterogenen Zellkultur-Fluid-Mischung zu reinigen, wurde eine Miniplant mit folgenden Modulen und zugehörigen Verfahrensschritten aufgebaut:
Wenn nicht anders vermerkt wurden im Prozess MasterFlex-Schlauchpumpen mit einem EasyLoad-II Pumpenkopf verwendet. Als Schläuche wurden Masterflex LS16 bzw. Cflex oder Sanipure verwendet. Alle eingesetzten produktberührten Bauteile wurden mit 25kGy Gamma-bestrahlt. In Ausnahmen, wenn Gammabestrahlung materialtechnisch nicht zulässig war, wurden Komponenten bei 121°C für 20min autoklaviert. Teilassemblies mit pH-Sonden oder Virenfilter. Wenn möglich wurden fertig einsetzbare Einwegartikel ("Disposables", "ready-to-use") als gammabestrahlte Module verwendet. Dies war ausnahmslos bei allen Beuteln ("Bags") der Fall. Diese Bags wurden in der Regel mit ReadyMate^{®} Konnektoren der Firma General Electric (GE) an die Module verbunden. Zwischen jedem Modul wurde eine Single-Use gammabestrahlte Bag (ReadyCircuit 1L, GE) als Ausgleichsbehälter zwischen dem Ausgangsstrom des Moduls n-1 und dem Eingangsstrom des Moduls n platziert. In der Regel existierte zu dem Zeitpunkt in jedem Modul ein Eingangs- und ein Ausgangsstrom. Wo eine Entlüftung der Produktflüssigkeit vorteilhaft war, wurden die Behälter über einen hydrophoben 0,2 µm Filter von der Umgebung abgeschlossen.

### A. Upstream

### i) Perfusions Reaktor

Zur kontinuierlichen Herstellung eines monoklonalen Antikörpers IgG wurde ein 10 L Perfusionsreaktor verwendet. Die viable Zelldichte betrug im stationären Zustand 60-70 Mio Zellen / mL. Der Titer betrug ∼115 mg/L . Die Produktion wurde mit zwei parallelen Perfusionsreaktoren für 28 Tage betrieben.

### ii) Zellrückhaltesystem System

Das Produkt wurde kontinuierlich über einen Schrägplattenabscheider (Settler) abgeführt, durch den ein Großteil der Zellen zurückgehalten wurde.

### B. Downstream DSP-1

### i) Zell-Klärfiltration

Die Klärfiltration erfolgte über parallel betriebene Sartoguard NF 0,2µm Filter (T-style, MaxiCap, 0,65m²). **Fig. 2** zeigt wie hier ein geschlossener keimarmer Prozess realisiert wurde. Sowohl die Filter wie auch das Schlauchassembly wurden gammabestrahlt. Die Eingangs- und Ausgangsleitungen wurden über aseptische Konnektoren mit gammabestrahlten Bags (GE ReadyCircuit 1 L) verbunden, die als Ausgleichsvolumina für schwankende Flussraten dienten. Zum Entlüften waren die Filter dabei an hydrophobe 0,2µm Luftfilter gekoppelt wodurch das Modul im Sinne der Erfindung geschlossen war **(****Fig.2****).** Der Luftfilter war entweder ein Emflon II der Pall Corp. oder eine Midisart2000 der Firma Sartorius Stedim. Die Entlüftungsventile waren dabei in der Art modifiziert, dass sie auch im geschlossenen Zustand durchgängig waren, dabei aber immer noch zuverlässig zur Umwelt hin abdichteten. Hierzu wurde am Sartoguard NF der innere Dichtungsring des Entlüftungsventils entfernt und das Ventil vor der Gammabestrahlung geschlossen. Dieses Ventil wurde gegen Öffnen zusätzlich gesichert. Das Ventil befand sich dadurch im sicheren Zustand "geschlossen", was durch einen festen Sitz charakterisiert war. Das Entlüftungsventil war mit dem Luftfilter über einen Schlauch verbunden. Zwischen Entlüftungsventil und Luftfilter war der Schlauch in ein Schlauchquetschventil eingelegt. Der Filter wurde befüllt, bis Flüssigkeit durch das Entlüftungsventil und den Schlauch in den Luftfilter eintrat. Der hydrophobe Entlüftungsfilter blockierte dann die Flüssigkeit. Gleichzeitig blockierte die Prozessanlage durch ein Ventil auf der Filtratseite den Produktionsstrom, so dass es zu einem Druckanstieg vor dem Filter kam. Dieser Druckanstieg wurde über einen Pendotech-Drucksensor detektiert. War ein Schwellenwert von 0,5 bar überschritten, wurde das Quetschventil des Entlüftungsschlauches geschlossen, und das Ventil auf der Filtratseite geöffnet.

### ii) Konzentration und Umpufferung

Das Filtrat aus der i) Zell-Klärfiltration wurde zunächst über eine Ultrafiltrations-Hohlfaser-Membran (GE Healthcare ReadytoProcess, 0,2m², gammabestrahlt) kontinuierlich um den Faktor 10 aufkonzentriert. Als Kreislaufpumpe diente eine disposable QuattroFlow 1200 SU Pumpe, dessen Pumpenkopf vor der Gammabestrahlung in das Schlauchassembly eingebunden wurde.

Die Medienbestandteile des konzentrierten Produkts wurde dann mit einem 50mM Imidazol/NaCl Puffer über eine Gambro Revaclear300 Dialysemembran ausgetauscht. Das Modul wird vom Hersteller steril verpackt geliefert und wurde unter einer Sterilbank mit dem gammabestrahlten Schlauchassembly verbunden. Das Permeat aus der Aufkonzentrierung und der medienbehaftete Abfallstrom wurden in einen gammabestrahlten 200L Sartorius Flexboy geleitet. Das Auswechseln des Flexboys wurde durch Umschweißen mit einem Sartorius Schweißgerät durchgeführt.

### 0,2µm Filtration

Das Produkt wurde vor der Abfüllung kontinuierlich mit gammabestrahlten Sartoguard NF Filtern (MaxiCap Size 8) im Wechselbetrieb in ein 200L Flexboy filtriert. Aufbau und Betrieb war Analog zu B. Downstream DSP-1 i) Zell Klärfiltration.

### C. Downstream DSP-II

### 0,2µm Filtration

Nach der Lagerung wurde das Produkt aus dem DSP-1 nochmals filtriert, um die nachgeschalteten Chromatographiesäulen vor Partikel zu schützen.

### 1. Capture Chromatography

Mabselect Sure (GE) wurde als Prot-A Harz zum Isolieren des IgG benutzt. Dabei wurde der IgG bis zu Faktor 10 aufkonzentriert und der größte Teil der Kontaminanten entfernt. Eine kontinuierliche BioSMB-Anlage der Firma Tarpon Biosystems, Inc. wurde benutzt mit 12 Säulen (ID 16 mm, L 80 mm), wobei 8 Säulen in der Ladezone waren (2 Säulen in Serie und 4 parallele Serien). Der gesamte Flussweg inklusive der Säulen wurde durch Sanitisierung oder gamma-Bestrahlung keimarm gemacht. Die Beladung pro Zyklus betrug 32 Säulenvolumina pro Säule. Als Puffer wurden Acetat Puffer mit unterschiedlichen Molarität, pH und Leitfähigkeiten eingesetzt. Sämtliche Puffer wurden über einen gammabestrahlten oder autoklavierten 0,2µm Filter in ein gammabestrahltes Bag filtriert. Die Ausgangsschläuche der Pufferbags wurden an die Eingänge der BioSMB-Anlage geschweißt. Diese hatte an Ihren Eingängen jeweils eine gammabestrahlte Entgaser Membran (Liquicell Micro Module, Membrana). In gleicherweise wurde die Produktleitung über einen solchen Entgaser an den Eingang der BioSMB-Anlage angeschweißt. Über eine Vakuumpumpe mit 50mbar wurden dann alle Eingangsströme entgast.

### 1. Virus-Inaktivierung und Neutralisierung

Das Virus-Inaktivierung und Neutralisierung bestand aus drei Modulen und befand sich zwischen der Capture Chromatography und einer 0,2µm Filtration: (a) eine Homogenisierungsschleife mit einer Schlauchpumpe M0502 (b) einer Verweilzeitschleife schematisch als gewickelter Schlauch dargestellt (c) einem Neutralisierungsbag in dem der pH auf 7,5 eingestellt werden konnte. Die Module wurden entsprechend der Schweißstellen in **Fig.3** einzeln vorgefertigt und gammabestrahlt, wobei die Enden jeweils zugeschweißt wurden.

Leitungsabschnitt mit pH Sonden, hier pH0501 und pH0502, wurden autoklaviert. pH Sonden Abschnitte wurden dann in Assemblies eingeschweißt.
Bags wurden wie gezeigt über aseptische GE ReadyMate^{®} Konnektoren angeschlossen. Die Verbindungen zur Protein A Eluat-Leitung und Filtrationsmodul waren zunächst zugeschweißt und wurden dann an die jeweiligen Module angeschweißt.

### 0,2µm Filtration

Proteine konnten nach einem pH-Shift präzipitieren, wobei das präzipitierte Protein abfiltriert wurde.

### Chromatographie Intermediat und Polish

Das Produkt aus der obigen 0,2µm Filtration wurde über zwei Chromatographieschritte durch zunächst vier sequentiell (4-PCC) betriebenen 2,5ml Capto Adhere (2,5ml GE) und dann zwei wechselseitig betriebenen 20ml Anionentauscher (Pall Hypercel StarAX) aufgereinigt. Dabei wurden ProtA Leachables, DNA, HCP und Aggregate abgereinigt. Die zwei Chromatographieschritte wurden über ein gammabestrahles Bag (GE ReadyCircuit^{®} 1 L) miteinander verbunden, in dem durch Zufuhr von Wasser die Leitfähigkeit entsprechend der Anforderung des Anionentauscher auf 7,5mS/cm eingestellt wurden. Der gesamte Flussweg inklusive der Säulen wurde sanitisiert oder gammabestrahlt. Die Beladung pro Zyklus betrug 50 Säulenvolumina pro Säule. Als Puffer wurden Acetat Puffer mit unterschiedlichen Molarität, pH und Leitfähigkeiten eingesetzt. Sämtliche Puffer wurden über einen gammabestrahlten oder autoklavierten 0,2µm Filter in ein gammabestrahltes Bag filtriert. Die Ausgangsschläuche der Pufferbags wurden an die Eingänge der BIO- geschweißt. Diese hatte an Ihren Eingängen jeweils eine gammabestrahlte Entgasungsmembran (Liquicell Micro Module, Membrana), In gleicherweise wurde die Produktleitung der obigen 0,2µm Filtration über einen solchen Entgaser an den Eingang der BioSMB-Anlage angeschweißt. Über eine Vakuumpumpe mit 50mbar wurden dann alle Eingangsströme entgast. Der Abfallstrom wurde in einen gammabestrahlten 200L Sartorius Flexboy geleitet. Das Auswechseln des Flexboys wurde durch Umschweißen mit einem Sartorius Schweißgerät durchgeführt.
Der Produktstrom wurde wiederum in einem gammabestrahlten Produktbag (GE ReadyCircuit 1 L) aufgefangen.

### Vorfiltration

Aus dem Produktbag der Polish Chromatographie wurde die Produktlösung zunächst über eine 0,1 µm Kapsule (Sartopore2, MidiCap size 9, 0,2m²) vorfiltriert. Durchführung und Aufbau waren analog zu der B. Downstream DSP-1 i) Zell Klärfiltration.

### Virus Filtration

Die Ausgangsleitung aus der Vorfiltration wurde direkt über eine Schlauchpumpe mit dem Eingang der Virusfiltration aus C. Downstream DSP-II über Verschweißen verbunden. Ansonsten war der Aufbau und Betrieb der Virusfiltration aus C. Downstream DSP-II analog zu C. Downstream DSP-II 0,2µm Filtration. Jedoch wurde als Virusfilter ein Virosart CPV Filter (MidiCap size 9, 0,2m²) verwendet, der gemäß der Herstellemorschrift eingespült und autoklaviert wurde. Wiederum wurden die Filter in das Assembly eingeschweißt. Der Produktstrom wurde wiederum in ein gammabestrahles Produktbag (GE ReadyCircuit 1 L) gepumpt.

### Finale Konzentrierung und Umpufferung

Die finale Konzentration und Umpufferung war analog zu der obigen B. DSP-I ii) Konzentration und Umpufferung aufgebaut und unterschied sich nur dadurch, dass eine autoklavierte UV-Zelle zur Überwachung der Produktkonzentration in die Konzentrationsschleife eingebunden war. Die Umpufferung erfolgte ebenfalls analog zu der obigen B. DSP-I ii) Konzentration und Umpufferung, wobei hier ein 50mM Phosphatpuffer pH 7,5 eingesetzt wurden. Der Produktstrom wurde wiederum in ein gammabestrahles Produktbag (GE ReadyCircuit 1 L) gepumpt.

### 0,2µm Filtration

Die finale Filtration erfolgte wie oben in B. DSP-1 i) über gammabestrahlte Sartopore 2 Kapsulen (Midicap size 7, 0,05m²) in ein gammabestrahltes 5L GE ReadCircuit Bag. Wenn der Füllstand des finalen Bags ausreichend war, wurde dieses Bag abgeschweißt und ein neues Bag an den Prozess angeschweißt.

Eine regelmäßig gefahrene Laufzeit des erfindungsgemäßen Verfahrens, wie in Beispiel 1 beispielhaft beschrieben, war 3 Tage ohne Keimwachstum, wobei die Chromatographiesäulen durch eine 40 % Isopropanol + 0.5 M NaOH sanitisiert wurden. Bei Laufzeiten des erfindungsgemäßen Verfahrens von über 3 Tage wurden die Chromatographiesäulen gamma -bestrahlt.

Die gemittelten Flussraten und Antikörperkonzentrationen der in **Figur 1** dargestellten Positionen sind in **Tabelle 1** zusammengefasst.

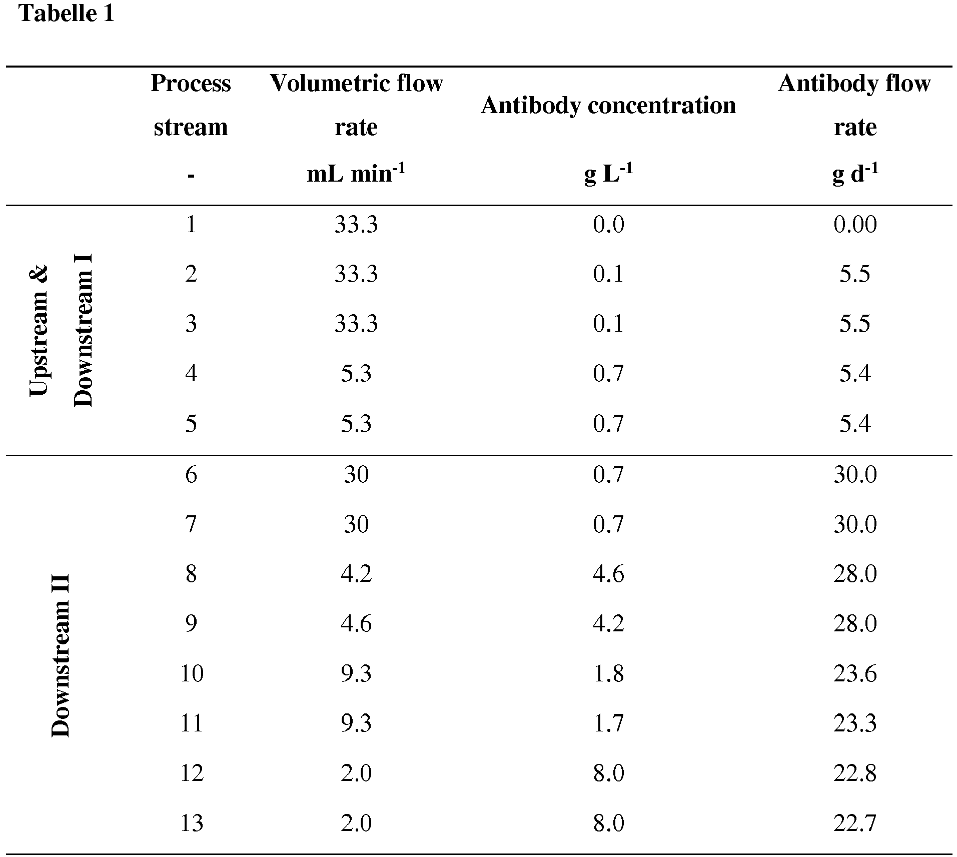

**Die Arbeiten, die zu dieser Anmeldung geführt haben, wurden gemäß der Finanzhilfevereinbarung "Bio.NRW: MoBiDiK - Modulare Bioproduktion - Disposable und Kontinuierlich" im Rahmen des Europäischen Fonds für regionale Entwicklung (EFRE) gefördert.**

## Patentansprüche

1. Verfahren zur kontinuierlichen, keimreduzierten Produktion und/oder Aufbereitung eines biopharmazeutischen, biologischen makromolekularen Produktes aus einer heterogenen Zellkultur-Fluid-Mischung, umfassend die Schritte:
(a) Bereitstellen eines partikel-freien Fluids aus einer heterogenen Zellkultur-Fluid-Mischung, welche das Produkt enthält, in Form eines Produktstroms,
(b) mindestens eine Filtration, wobei ein Filtrat erhalten wird,
(c) wenigstens zwei Chromatographieschritte zur Reinigung des Produktes,
(d) mindestens eine Virenabreicherung,
(e) mindestens eine Ultrafiltration und/oder mindestens eine Diafiltration des Produktstroms (26) der Schritte (b), (c), und/oder (d),
**dadurch gekennzeichnet, dass** die wenigstens zwei Chromatographieschritte aus (c) eine Reinigung über wenigstens jeweils zwei Chromatographiesäulen (4) und/oder Membranadsorber (5) umfasst und,
dass das Verfahren geschlossen und modular durchgeführt wird.

2. Verfahren nach Anspruch 1, **dadurch gekennzeichnet, dass** zwischen den wenigstens zwei Chromatographieschritten in (c) und/oder nach der Vireninaktivierung in Schritt (d) ein oder mehrere weitere Schritte zur Einstellung des pH-Wertes und/oder der Leitfähigkeit und/oder Filtrations- und/oder Konzentrationsschritte und/oder ein Pufferaustausch erfolgen.

3. Verfahren nach Anspruch 1 oder 2, **dadurch gekennzeichnet, dass** alle in den Schritten (a) bis (e) eingesetzten, produkt-berührten Elemente durch eine geeignete Keimreduktionsmethode keimreduziert sind, wobei die Keimreduktionsmethode bevorzugt ausgewählt ist aus der Gruppe bestehend aus Gamma-Bestrahlung, Beta-Bestrahlung, Autoklavieren, Ethylenoxid (ETO)-Behandlung, Ozon-Behandlung (O₃), Wasserstoffperoxid-Behandlung (H₂O₂) und Steam-in-place (SIP) Behandlung.

4. Verfahren nach Anspruch 1 bis 3, **dadurch gekennzeichnet, dass** alle ab Schritt (b) verwendeten produktberührten Elemente Einwegartikel sind oder als Einwegartikel benutzt werden.

5. Verfahren nach Anspruch 1 bis 4, **dadurch gekennzeichnet, dass** alle Eingangsfluide durch einen Keimreduktionsfilter (10) filtriert werden und, dass bevorzugt alle Ausgänge durch eine Keimbarriere (11) geschützt sind, die ein Rückwachstum verhindern.

6. Verfahren nach Anspruch 1 bis 5, **dadurch gekennzeichnet, dass** die modularen Verfahrensschritte in Modulen durchgeführt werden, wobei die Module miteinander verbunden sind, wobei die Module bevorzugt durch Verschweißen oder durch aseptische Konnektoren (12) miteinander verbunden sind.

7. Verfahren nach einem der Ansprüche 1 bis 6, **dadurch gekennzeichnet, dass** in den Schritten (a) bis (e) alle verwendeten Flüssigkeiten, Gase und Feststoffe keimreduziert sind, wobei die Keimreduktion bevorzugt durch eine Filtration durch einen Filter (13) mit einer Porengröße von bevorzugt ≤ 0,45 µm erfolgt, und, dass bevorzugt während des Verfahrens keine Inprocess-Stelilisierung durchgeführt wird.

8. Verfahren nach Anspruch 1 bis 7, **dadurch gekennzeichnet, dass** vor Schritt (c) ein Entgasen aller Fluide durchgeführt wird, welche auf die wenigstens zwei Chromatographiesäulen (4) kommen, wobei dass Entgasen vorzugsweise durch mindestens eine Bubble-Trap (14) und/oder durch mindestens eine hydrophobe Mikrofiltrationsmembran (15) über Vakuum und/oder durch Behandeln mit Ultraschall und/oder durch Durchperlen mit Helium erfolgt.

9. Verfahren nach Anspruch 1 bis 8, **dadurch gekennzeichnet, dass** das partikel-freie Fluid aus Schritt a) mindestens einer Ultrafiltration gegen einen mikrobizid-haltigen Puffer unterzogen wird, wobei das Mikrobizid bevorzugt ausgewählt ist aus der Gruppe bestehend aus Imidazol, Benzoesäure, Sorbinsäure, para-Hydroxybenzoesäure-Ester, Sulfite, Disulfite, Azide, Orthophenylphenol, Nisin, Natamycin, Hexamethylentetramin, Dimethyldicarbonat, Nitrite, Nitrate, Essigsäure, Ascorbinsäure, Isoascorbinsäure, L-Milchsäure, Propionsäure, Borsäure und Lysozym.

10. Verfahren nach einem der Ansprüche 1 bis 9, **dadurch gekennzeichnet, dass** das biopharmazeutische, biologische makromolekulare Produkt ein Protein, Peptid ist oder eine DNA oder RNA umfasst, wobei das Protein oder Peptid ausgewählt ist aus der Gruppe bestehend aus monoklonalen Antikörpern, polyklonalen Antikörpern, rekombinanten Proteinen und Protein-Impfstoffen, und wobei die DNA oder RNA Teil eines DNA- und/oder RNA-Impfstoffes ist.

11. Verfahren nach einem der Ansprüche 1 bis 10, **dadurch gekennzeichnet, dass** die wenigstens zwei Chromatographiesäulen (4) und/oder Membranadsorber (5) des Schrittes (c) Produkt nach dem Affinitätsprinzip, über ionische Wechselwirkungen, über Metalchelat-Bindung, über hydrophobe Interaktionen oder über van-der-Waals-Kräfte binden, wobei die wenigstens zwei Chromatographiesäulen (4) und/oder Membranadsorber (5) bei Bindung nach dem Affinitätsprinzip einen Ligand umfassen, der bevorzugt ausgewählt ist aus der Gruppe bestehend aus Protein A, Protein G, Protein L, IgM, IgG und einem von Protein A, Protein G, Protein L, IgM und IgG unterschiedlichen rekombinanten Protein, welches eine Affinität für das Produkt hat.

12. Verfahren nach einem der Ansprüche 1 bis 11, **dadurch gekennzeichnet, dass** das Verfahren der Schritte (a) bis (e) eine Laufzeit von mindestens 4 Stunden, bevorzugt von mindestens 8 Stunden, bevorzugt von mindestens 12 Stunden, bevorzugt von mindestens 24 Stunden, weiterhin bevorzugt von mindestens 48 Stunden, weiterhin bevorzugt von mindestens 7 Tagen, weiterhin bevorzugt von mindestens 4 Wochen, und besonders bevorzugt von mindestens 8 Wochen aufweist.

13. Verfahren nach einem der Ansprüche 1 bis 12, **dadurch gekennzeichnet, dass** zwischen den Schritten a) bis e) und/oder danach mindestens ein Filtrationsschritt umfassend mindestens einen Filter (13) durchgeführt wird.

14. Verfahren nach Anspruch 13, **dadurch gekennzeichnet, dass** der Filter (13) automatisch unter keimreduzierten Bedingungen gewechselt wird, wobei bevorzugt der automatische Filterwechsel die folgenden Schritte umfasst:
(i) Umschalten des Flussweges auf einen neuen Filter (16) bei Überschreiten eines Schwellenwertes an einem Drucksensor (18) auf der Unfiltratseite unter Verschließen des Flussweges, wobei das Produkt im verbrauchten Filter (17) bevorzugt durch ein Gas oder eine Flüssigkeit in die Filtratseite gedrückt wird, oder bei Überschreiten einer maximalen Zeit des verbrauchten Filters (17) im Flussweg, oder bei Überschreiten eines maximalen Volumens an Filtrat durch den verbrauchten Filter (17),
(ii) Entlüften des neuen Filters (16) über einen Luftfilter (19) mit einer Porengröße von bevorzugt <= 0,25 µm am Entlüftungsventil (20) des neuen Filters (16), bevorzugt unter Beförderung von Produkt in den neuen Filter (16) durch eine Feedpumpe (21), oder in einen keimreduziert angeschlossenen, geschlossenen Bag (29),
(iii) Detektion der Beendigung der Entlüftung des neuen Filters (16) auf der Unfiltratseite durch den Drucksensor (18) oder einen Füllstandssensor (22) oder eine Waage (23) oder einen Flüssigkeitsdetektor (28),
(iv) Öffnen des Filtratausgangs und Verschluss des Flussweges zwischen Entlüftungsventil (20) und Luftfilter (19) durch ein Ventil (24), und
(v) Austausch des verbrauchten Filters (17) gegen einen neuen Filter (16).

15. Modulare Anlage (1) zur kontinuierlichen, keimreduzierten Produktion und/oder Aufbereitung eines biopharmazeutischen, biologischen makromolekularen Produktes aus einer heterogenen Zellkultur-Fluid-Mischung, umfassend folgende Module:
(a) mindestens ein Filtrationsmodul (2),
(b) mindestens ein Chromatographiemodul (3), umfassend mindestens jeweils zwei Chromatographiesäulen (4) und/oder Membranadsorber (5),
(c) mindestens ein Ultrafiltrationsmodul (6) und/oder mindestens ein Diafiltrationsmodul (7) und/oder mindestens ein Dialysemodul (8), und
(d) mindestens ein Modul zur kontinuierlichen Virenabreicherung (9),
**dadurch gekennzeichnet, dass** die modulare Anlage (1) geschlossen und keimreduziert ist.

16. Modulare Anlage (1) nach Anspruch 15, **dadurch gekennzeichnet, dass** alle in den Modulen (a) bis (d) eingesetzten und mit produktberührten Elemente durch eine Keimreduktionsmethode keimreduziert sind, wobei die Keimreduktionsmethode bevorzugt ausgewählt ist aus der Gruppe bestehend aus Gamma-Bestrahlung, Beta-Bestrahlung, Autoklavieren, Ethylenoxid (ETO)-Behandlung, Ozon-Behandlung (O₃), Wasserstoffperoxid-Behandlung (H₂O₂) und steam-in-place (SIP) Behandlung.

17. Modulare Anlage nach Anspruch 15 oder 16, **dadurch gekennzeichnet, dass** alle in den Modulen (a) bis (d) verwendeten produktberührten Elemente Einwegartikel sind oder als Einwegartikel benutzt werden und, dass die Module bevorzugt durch Verschweißen oder durch aseptische Konnektoren miteinander verbunden sind.

18. Modulare Anlage nach Anspruch 15 bis 17, **dadurch gekennzeichnet, dass** alle Eingangsfluide durch einen Keimreduktionsfilter (10) gehen und, dass bevorzugt alle Ausgänge durch eine Keimbarriere (11) geschützt sind, die ein Rückwachstum von Mikroorganismen verhindern.
